# EUROPÄISCHE PATENTSCHRIFT

(11) **EP 1 411 050 B1**
(45) Veröffentlichungstag und Bekanntmachung des Hinweises auf die Patenterteilung: **14.03.2007**
(21) Anmeldenummer: 03018964.1
(22) Anmeldetag: 21.08.2003
(51) Int. Cl.: C07D 301/12, C07D 303/04

(54) **Verfahren zur Epoxidierung cyclischer Alkene**
Process for the epoxydation of cycloalkenes
Procédé de l'époxydation des cycloalcenes

(30) Priorität: 11.10.2002 DE 10247495
(43) Veröffentlichungstag der Anmeldung: 21.04.2004
(73) Patentinhaber: Degussa GmbH, 40474 Düsseldorf (DE)
(72) Erfinder: Herwig, Jürgen, Dr., 46565 Hünxe (DE); Roos, Martin, Dr., 45721 Haltern am See (DE); Oenbrink, Georg, Dr., 48249 Dülmen (DE); Günzel, Bernd, Dr., 45721 Haltern am See (DE); Lohmar, Jörg, Dr., 44141 Dortmund (DE)

(56) Entgegenhaltungen:
- DE-A- 3 027 349

## Beschreibung

Die vorliegende Erfindung bezieht sich auf ein Verfahren zur Epoxidierung von cyclischen Alkenen mit 8 - 20 Kohlenstoffatomen durch katalytische Oxidation, in Gegenwart einer Peroxoverbindung als Oxidationsmittel und eines heterogenen und/oder homogenen Katalysators, in flüssiger Phase. Sie bezieht sich insbesondere auf ein Verfahren zur Herstellung von Epoxiden durch Oxidation von Alkenen in flüssiger Phase mit Wasserstoffperoxid in Anwesenheit eines Katalysatorsystems auf Basis eines Übergangsmetalles.

Es sind zahlreiche Verfahren zur Epoxidierung von Alkenen bekannt, und eine große Bandbreite verschiedener Reaktions- beziehungsweise Katalysatorsysteme kann eingesetzt werden. Die Epoxidierung von Alkenen in homogener, flüssiger Phase mit organischen Hydroperoxiden in Anwesenheit von Katalysatoren auf der Basis Molybdän, Wolfram beziehungsweise Vanadium findet industrielle Anwendung.
Die Epoxidherstellung wird jedoch durch äquivalente oder sogar größere Mengen des dem Hydroperoxid entsprechenden Alkohols begleitet, dessen Verwertung oder Rückführung eine großtechnische Anwendung dieses Verfahrens stark einschränkt.

Aus diesem Grund wurden verstärkt direktere Oxidationsverfahren (Epoxidierung) von Alkenen entwickelt.

Ein entsprechendes Verfahren ist die Epoxidierung mittels molekularem Sauerstoff unter Verwendung von Silberkatalysatoren. Dieses Verfahren konnte aber nur bei Ethen erfolgreich angewendet werden; es ließ sich nicht auf andere, interessante Alkene (wie zum Beispiel Propen) analog übertragen.

Ein anderes Verfahren für eine direkte Oxidation von Alkenen zu Epoxiden ist die Umsetzung mit Wasserstoffperoxid.
Dieses Verfahren ist vor allem aufgrund der positiven Eigenschaften des Oxidationsmittels in Bezug auf eine wesentlich geringere Umweltbelastung für verschiedene Epoxidationsverfahren vorgeschlagen worden. Da die Aktivität von Wasserstoffperoxid gegenüber den Alkenen nur gering ist, teilweise sogar völlig fehlt, müssen Aktivierungsmittel, gewöhnlich organische Säuren, wie Ameisensäure, Essigsäure usw., in organischen Lösemitteln verwendet werden, wobei die Säuren in Form von Persäuren das reaktionsfähige Epoxidationsmittel in situ bilden.

Auch diese Verfahren scheinen nicht besonders erfolgreich, und zwar weil es schwierig ist, die Persäuren zu erhalten und aufgrund der Instabilität der Epoxide in saurem Medium, wodurch recht mühsame Verfahrensbedingungen notwendig werden.
Ein weiteres Verfahren ist die Oxidation von Alkenen durch Reaktion mit hoch konzentriertem Wasserstoffperoxid in homogener, d. h. ausschließlich organischer, flüssiger Phase in Anwesenheit löslicher Katalysatorsysteme auf der Basis von Elementen der Gruppen 4, 5 und 6 des Periodensystems (Ti, V, Mo, W) in Verbindung mit Elementen aus der Gruppe von Pb, Sn, As, Sb, Bi, Hg usw.
Auch hier lassen die Ergebnisse keine Übertragung auf ein industrielles Verfahren zu. Denn einerseits läuft die Reaktion langsam ab, andererseits ist die Herstellung der Katalysatorsysteme, die im Allgemeinen aus sehr komplizierten organischen Metallverbindungen bestehen und darüber hinaus in dem organischen Reaktionsmedium löslich sein müssen, aufwändig und teuer. Des weiteren bringt die Anwendung von hoch konzentriertem Wasserstoffperoxid (> 70 %) erhebliche Sicherheitsgefahren mit sich, die in wirtschaftlicher Weise nicht leicht überwunden werden können.

Diese Verfahren des Standes der Technik verdeutlichen, dass die Oxidation von Alkenen mit Wasserstoffperoxid in sich selbst widersprüchlich ist, weil die Arbeitsbedingungen bezüglich Katalysatorsystem und Wasserstoffperoxid bestenfalls ein wässriges, möglichst saures Medium erfordern, während die Oxidationsreaktion und die Stabilität des Epoxids vorzugsweise ein neutrales organisches Medium benötigen.

Daher wurden weitere Verfahren zur Epoxidierung von Alkenen mit Wasserstoffperoxid entwickelt, bei denen entweder ein verbessertes Katalysatorsystem auf Basis von TiO₂/SiO₂ in wässriger Phase unter Zusatz von primären oder sekundären Alkoholen (vgl. EP 0 987 259 A1) oder ein Zweiphasensystem mit einem Katalysator aus Wolframsäure, einem quartären Ammoniumsalz und einer Phosphorverbindung (zum Beispiel DE 30 27 349) zum Einsatz kommt.

Für Alkene, deren Epoxide nicht hydrolyselabil sind und bei denen die olefinische Doppelbindung nicht sterisch gehindert ist (wie zum Beispiel cyclische mindestens einfach ungesättigte Alkene) ist die bekannte Epoxidierung mit Wasserstoffperoxid und einem Wolframkatalysator die wirtschaftlichste Alternative.

Damit die Epoxidierung mit Wasserstoffperoxid schnell genug verläuft, wird bei sehr lipophilen Alkenen (wie zum Beispiel Cyclododecen) meist ein Phasentransferkatalysator (zum Beispiel Aliquat^{®} 336) verwendet (Angew. Chem. (1991), 103(12), 1706 - 9). Die gewünschte starke Beschleunigung der Epoxidierung durch den Phasentransferkatalysator führt jedoch dazu, dass die Phasen nach der Reaktion aufgrund von Emulsionsbildung deutlich schwieriger zu trennen sind; die entsprechenden Absetzzeiten erhöhen sich stark. Auch verbleibt die organische Phase nach der Trennung meistens stark getrübt. Um eine weitgehend vollständige Phasentrennung zu erreichen, müssen entweder Phasentrenner mit sehr großen Volumina oder geeignete Zentrifugen eingesetzt werden.

Durch die erhöhten Absetzzeiten dieses Verfahrens wird die Attraktivität für eine großtechnische, kontinuierliche Anwendung stark vermindert. Insbesondere ist eine Übertragung des Verfahrens auf bestehende Anlagen aufgrund der Notwendigkeit vergrößerter Phasentrenner meistens aufgrund von Platzproblemen gar nicht realisierbar. Die Verwendung von Zentrifugen ist angesichts der Stromkosten und des Wartungsaufwandes aufgrund bewegter Teile uninteressant.

Ganz allgemein kann gesagt werden, dass Absetzzeiten von weniger als 2 Minuten als technisch wünschenswert zu bezeichnen sind. Betragen die Absetzzeiten dagegen mehr als 4 Minuten, so kann ein großtechnischer kontinuierlicher Prozess nur schwer wirtschaftlich betrieben werden.

In der DE 30 27 349 wird ein Verfahren zur Epoxidierung von Alkenen mit Wasserstoffperoxid, einer Wolframverbindung, einer Phosphorverbindung und einem Phasentransferkatalysator beschrieben. Bei diesem Verfahren werden zwingend Lösemittel verwendet, beispielsweise Alkane oder Cycloalkane. Diese Lösemittel werden immer in größeren Mengen der Reaktionsmischung zugesetzt und dienen im Allgemeinen dazu, entweder einen festen Stoff aufzulösen und so zur Reaktion zu bringen, oder die Reaktionsführung zu verbessern, beispielsweise um eine bessere Wärmeabfuhr zu gewährleisten.

Allerdings ist die Verdünnung von Edukten mit nicht reaktionsfähigen Stoffen, wie zum Beispiel Lösemitteln, nicht erwünscht, da einerseits die Verdünnung zu einer Erniedrigung der Raum-Zeit-Ausbeute führt und anderseits eine weitere Trennoperation nach der Reaktion erforderlich ist.

Die Aufgabe der vorliegenden Erfindung besteht in der Bereitstellung eines Verfahrens zur Epoxidierung von Alkenen, das kontinuierlich betrieben werden kann, das zu technisch akzeptablen Absetzzeiten der heterogenen Katalysator/Reaktionsmischung führt und das eine ausreichend hohe Raum-Zeit-Ausbeute an Epoxid gewährleistet, so dass es für eine großtechische Herstellung entsprechender Epoxide übertragbar ist.
Darüber hinaus soll dieses Verfahren unter Verwendung bestehender Anlagen zur Epoxidierung von Alkenen angewendet werden können, ohne dass größere Umbauten vorgenommen werden müssen.

Die Aufgabe wird erfindungsgemäß gelöst durch ein Verfahren zur Epoxidierung von cyclischen, mindestens einfach ungesättigten Alkenen mit 8 - 20 Kohlenstoffatomen in Gegenwart einer Peroxoverbindung als Oxidationsmittel und eines heterogenen und/oder homogenen Katalysators, wobei die Reaktionsmischung mindestens 1 Gew.-% des korrespondierenden gesättigten Alkans enthält.
In einer besonders bevorzugten Ausführungsform enthält das Reaktionsgemisch mindestens 2,5 Gew.-% des zum eingesetzten cyclischen Alkens korrespondierenden Alkans.

Die Angabe "Gew.-%" bedeutet im gesamten Anmeldetext der in Prozenten ausgedrückte, gewichtsmäßige Anteil der entsprechenden Komponente, bezogen auf das Epoxid.

Das Ergebnis des erfindungsgemäßen Gegenstands ist insofern überraschend, als dass die Anwesenheit des zum eingesetzten Olefins korrespondierenden Alkans bereits in geringen Mengen zu einer erheblichen Beschleunigung der Absetzzeiten und damit insgesamt zu einem wirtschaftlicheren Prozess führt.

Im Gegensatz zu den Verfahren des Standes der Technik, die einen Phasentransferkatalysator verwenden, führt das erfindungsgemäße Verfahren zu Absetzzeiten der am Ende der Oxidationsreaktion resultierenden heterogenen Mischung aus Katalysator und Produkt von weniger als zwei Minuten.
Unter dem Begriff Absetzzeit wird die Zeit verstanden, nach der sich die Phasen vollständig getrennt haben. Die Phasentrennung wird definitionsgemäß als beendet angesehen, wenn die letzte Tropfenschicht nur noch die Hälfte der Phasengrenze bedeckt, die andere Hälfte als klare Grenzfläche frei von Dispersionströpfchen zu erkennen ist (vgl.: "Dimensionierung liegender Flüssig-Flüssig-Abscheider anhand diskontinuierlicher Absetzversuche", Dipl. Ing. Martin Henschke, VDI-Verlag, Düsseldorf, 1995).

Darüber hinaus wird bei dem erfindungsgemäßen Verfahren nur das zu dem als Edukt eingesetzten Alken korrespondierende Alkan zugesetzt.
Auch wenn die Obergrenze des korrespondierenden Alkans an sich nicht für die Verringerung der Absetzgeschwindigkeit entscheidend ist, wird bevorzugterweise für die Lösung der Aufgabe nur eine geringe Menge des korrespondierenden Alkans verwendet, im Allgemeinen nicht mehr als 10 Gew.-%, insbesondere um eine akzeptable Raum-Zeit-Ausbeute zu gewährleisten. Das korrespondierende Alkan fungiert erfindungsgemäß nicht als Lösemittel.
Gemäß einer besonders bevorzugten Ausführungsform der vorliegenden Erfindung sind die Mengen jedoch so klein, dass die Raum-Zeit-Ausbeute der Epoxidierung nur unwesentlich verringert wird.

Eine weitere, ebenfalls bevorzugte Form des erfindungsgemäßen Verfahrens besteht darin, dass das heterogene Verfahren kontinuierlich betrieben wird.
Als Oxidationsmittel können alle dem Fachmann bekannten, Peroxoverbindungen, eingesetzt werden. Besonders bevorzugt ist als Oxidationsmittel Wasserstoffperoxid.
Sofern zur verbesserten Reaktionsführung ein Phasentransferkatalysator benötigt wird, ist der Einsatz oder die Gegenwart eines Katalysatorsystems, das mindestens ein Metall der Gruppen IVb, Vb oder VIb des Periodensystems der Elemente darstellt oder enthält, erfindungsgemäß bevorzugt. Weitere Bestandteile des Katalysatorsystems können Phosphorsäure und mindestens ein tertiäres Amin und/oder ein quartäres Ammoniumsalz sein. Es können aber auch je nach gewünschter Reaktionsführung Mischungen dieser Katalysatorsysteme verwendet werden. Bei Verwendung eines solchen Katalysatorsystems wird der Reaktionsmischung zur optimalen Reaktionsführung vorzugsweise Phosphorsäure und mindestens ein quartäres Ammoniumsalz hinzugegeben.

Der für das erfindungsgemäße Verfahren bevorzugt eingesetzte Katalysator ist ein Metall der Gruppen IVb, Vb oder VIb des Periodischen Systems, das in metallischer Form oder in Form eines Komplexes entsprechend dem Oxidationsgrad Null eingesetzt werden kann, oder in dem es sich mit einem variablen Oxidationsgrad befindet. Besonders bevorzugt werden Molybdän, Wolfram, Vanadium, Chrom und Titan.

Unter den anorganischen Derivaten dieser Elemente kann man die Oxide, die gemischten Oxide, die Hydroxide, Oxysäuren, Heteropolysäuren, deren Salze und Ester, die Salze, die von Wasserstoffsäuren und von anorganischen Oxysäuren und organischen Carboxyl- oder Sulfonsäuren mit nicht mehr als 20 Kohlenstoffatomen stammen, deren Anionen unter den Reaktionsbedingungen stabil sind, verwenden.
Als Beispiele für entsprechende Katalysatoren sind zu nennen: Molybdän, Wolfram, Chrom, Vanadium, Titan, die carbonylierten Metalle Mo(CO)₆, W(CO)₆, die Oxide MoO₂, Mo₂O₅, Mo₂O₃, MoO₃, WO₂, W₂O₅, WO₆, CrO₂, Cr₂O₃, CrO₃, VO₂, V₂O₅, ZrO₂, TiO, TiO₂, Ti₂O₃, NbO₂, Nb₂O₃, Nb₂O₅, die Sulfide MoS₂, MoS₃, MoS₄, Mo₂S₃, Mo₂S₅, die Oxychloride von Molybdän, Wolfram, Chrom, Vanadium, Zirkonium, Titan, die Fluoride, Chloride, Bromide, Jodide, Nitrate, Sulfate, Phosphate, Pyrophosphate, Polyphosphate, Borate, Carbonate, Formiate, Octanoate, Dodecanoate, Naphthenate, Stearate, Oxalate, Succinate, Glutarate, Adipate, Benzoate, Phthalate, Benzolsulfonate von Molybdän, Wolfram, Titan, Chrom, Zirkonium, Vanadium, Komplexe wie die Acetylacetonate und Phthalocyanine; die Molybdän-, Wolfram-, Vanadin-, Chromsäuren, die entsprechenden Heteropolysäuren, wie die Phosphormolybdän-, -wolfram-, Arsenmolybdän-, -wolframsäuren ebenso wie alle Alkali- oder Erdalkalisalze dieser Säuren.

In einer besonders bevorzugten Ausführungsform der vorliegenden Erfindung wird ein Wolframkatalysator unter Zusatz von Phosphorsäure verwendet. Die Wolframverbindung wird bevorzugt in einer Konzentrationen von 0,01 bis 0,5 Mol-%, bezogen auf das Alken, eingesetzt.
Der Anteil an Phosphorsäure beträgt üblicherweise 0,1 bis 5 Mol-%, bezogen auf die molare Menge an eingesetztem Wolfram.

Die verwendeten quartären Ammoniumsalze entsprechen der allgemeinen Formel

[NR₁R₂R₃R₄]⁺X⁻

wobei R₁, R₂, R₃ und R₄, unabhängig voneinander, lineare oder verzweigte Alkylketten mit 1 bis 20 Kohlenstoffatomen oder Arylgruppen mit 6 bis 10 Atomen darstellen. Die Alkyl- oder Arylgruppen können optional mit weiteren organischen Gruppen oder Atomen, wie zum Beispiel Halogenen, substituiert sein. X⁻ ist ein Gegenion für das Ammoniumion, wie zum Beispiel Chlorid, Bromid, Fluorid, Iodid, Hydrogensulfat, Acetat, Propionat oder Formiat.
Die tertiären Amine haben 3 Alkylreste, die - gleich oder verschieden - in Summe mindestens 18 Kohlenstoffatome aufweisen.

Im Allgemeinen wird als Katalysator bevorzugt ein homogener Katalysator verwendet. Es ist aber auch möglich, einen heterogenen Katalysator zu verwenden, bei dem die katalytisch wirksamen Bestandteile auf einem Trägermaterial, wie beispielsweise Aluminiumoxid, Siliziumdioxid, Aluminiumsilikat, Zeolithen oder geeigneten Polymeren in an sich bekannter Weise aufgebracht sind.
Die besten Ergebnisse im Hinblick auf das beanspruchte Verfahren wurden mit einem Katalysator erhalten, der in DE 30 27 349 beschrieben ist. Dieser Katalysator besteht aus einer ersten Komponente aus mindestens einem Element oder mindestens einem seiner anorganischen, organischen oder metallorganischen Derivate aus der Gruppe von W, Mo, V, vorzugsweise W, das in situ und bei Reaktionsbedingungen in eine katalytisch aktive Verbindung umgewandelt werden kann.
Ein besonders geeignetes System besteht aus 0,2 mol % Natriumwolframat bezogen auf Cycloalken, 0,2 mol % Phasentransferkatalysator Trioctylmethylammoniumchlorid bezogen auf Cycloalken, 0,1 mol % Phosphorsäure bezogen auf Cycloalken, und einer Menge Schwefelsäure, die den pH-Wert auf 3 einstellt.

Gemäß einer weiteren, ebenfalls bevorzugten Ausführungsform enthält das Reaktionsgemisch mindestens zwei flüssige Phasen. Davon ist die eine Phase eine wässrige Phase, in der das Wasserstoffperoxid gelöst ist. Dadurch entfällt der Einsatz polarer Lösemittel und der damit einhergehende Verlust an Raum-Zeit-Ausbeute sowie der erhöhte Trennungsaufwand.

Der pH-Wert sollte gemäß einer weiteren, bevorzugten Ausführungsform während der Reaktion konstant gehalten werden. Dies kann durch eine automatische pH-Wert Regelung erreicht werden. Als günstiger pH-Bereich wird erfindungsgemäß ein pH-Wert zwischen 2 und 6 gesehen. Besonders bevorzugt wird die Reaktion bei einem pH-Wert zwischen 2,5 und 4 durchgeführt.

Als cyclisches, mindestens einfach ungesättigtes Alken werden cyclische Alkene mit 8 bis 20 Kohlenstoffatomen im Cyclus eingesetzt.

Diese Alkene können gegebenenfalls durch funktionelle Gruppen substituiert sein, die in dem Reaktionsmilieu stabil sind, wie zum Beispiel Hydroxy-, Chlor-, Fluor-, Brom-, Iod-, Nitro-, Alkoxy-, Amino-, Carbonyl-, Säure-, Ester-, Amid- oder Nitril-Reste.
Sie können aber auch mehrfach ungesättigt sein, wie zum Beispiel Diene oder Triene in konjugierter oder nichtkonjugierter Form.

Besonders bevorzugt sind insbesondere Cycloocten, Cyclooctadien, Cyclododecen, Cyclododecadien, Cyclododecatrien, Dicyclopentadien und Cyclododecen zu nennen. Vor allem solche Alkene mit mehr als neun Kohlenstoffatomen lassen sich im Hinblick auf die Raum-Zeit-Ausbeute wesentlich einfacher umsetzen, verglichen mit den Verfahren des Standes der Technik.
Ganz besonders bevorzugt ist das Verfahren zur Herstellung cyclischer Epoxyalkane, insbesondere zur Herstellung von 1,2-Epoxycyclododecan.

Unter dem zum eingesetzten cyclischen, mindestens einfach ungesättigten Alken korrespondierenden Alkan versteht man die cyclische, gesättigte organische Verbindung mit der gleichen Anzahl von Kohlenstoffatomen. Die korrespondierenden Verbindungen sind dann beispielsweise ausgewählt aus der Gruppe von Cyclooctan, Cyclododecan und Dicyclopentan.

Das erfindungsgemäße, kontinuierliche Verfahren kann in einem oder mehreren Reaktoren durchgeführt werden. Ein Beispiel für eine kaskadenartige Reaktionsführung über mehrere Stufen wird in der EP 1 167 334 A2 beschrieben. Dort wird die Reaktionsmischung in die vordere Reaktionszone eingebracht und durchläuft mehrere nachgeschaltete Reaktionszonen, bis die das Produkt enthaltende Mischung schließlich an der letzten Reaktionszone abgeführt wird. Dabei können die einzelnen Reaktionszonen in unterschiedlichen Reaktoren oder in einem Reaktor integriert sein.

Eine besonders bevorzugte Ausfiihrungsform des erfindungsgemäßen Verfahrens unter Verwendung eines Phasentransferkatalysators ist, die Ausgangsmischung aus Alken und korrespondierendem Alkan zusammen mit dem quartären Ammoniumsalz in einen ersten Reaktor zu dosieren. In diesen Reaktor wird eine dem Alken entsprechende molare Menge an Wasserstoffperoxid dosiert, welches bereits Phosphorsäure und den in der DE 30 27 349 beschriebenen Katalysator auf Wolframbasis enthält. Es ist möglich, die Menge von Wasserstoffperoxid um 20 % zu über- oder zu unterschreiten. Ab einem gewissen Füllungsgrad des ersten Reaktors, der einer bestimmten Verweilzeit entspricht, wird die Lösung entweder über Überlauf, oder über eine Pumpe kontinuierlich in einen weiteren Reaktor gefördert. In diesen zweiten Reaktor wird gegebenenfalls eine kleine Menge Wasserstoffperoxid ohne Katalysator und Phosphorsäure dosiert. Ab einem gewissen Füllungsgrad des zweiten Reaktors, der einer bestimmten Verweilzeit entspricht, wird die Lösung entweder über Überlauf, oder über eine Pumpe kontinuierlich in einen weiteren Reaktor gefördert. In diesen dritten Reaktor wird gegebenenfalls einen kleine Menge Wasserstoffperoxid ohne Katalysator und Phosphorsäure dosiert. Diese Form der Reaktorkaskade kann über 2 bis 8 Stufen geführt werden. Diese Stufen können auch einen Reaktor integriert werden, in dem die Mehrstufigkeit des Reaktors über entsprechende Einbauten realisiert wird.
Das Wasserstoffperoxid kann je nach angestrebtem Umsatz auf die einzelnen Reaktoren aufgeteilt werden. Das verwendete Wasserstoffperoxid ist handelsübliches Wasserstoffperoxid mit Konzentrationen von 10 bis 70 % Wasserstoffperoxid in Wasser.

Nach beendeter Reaktion und vollständiger Phasentrennung kann das Wolfram aus der organischen Phase durch, optional mehrfache, Extraktion mit Wasser, welches gegebenenfalls basische Zusätze, wie zum Beispiel Natriumcarbonat, Natriumhydrogencarbonat oder Natriumhydroxid, enthält, extrahiert werden. Danach kann das Alkan, zusammen mit Resten nicht umgesetzten Alkens abdestilliert werden. Aus dem Sumpf dieser Destillation wird das reine Epoxid durch Feindestillation gewonnen. Die Vorbehandlung dient einer Reinigung vor der Destillation, um so die Entstehung von Nebenprodukten zu verringern.

Die Temperatur bei der Umsetzung kann zwischen 50 und 120 °C liegen. Bevorzugt sind Temperaturen zwischen 70 und 100 °C.

Die Durchführung der Reaktion wird bevorzugt unter Schutzgas (Stickstoff, Argon, Kohlendioxid) durchgeführt.

Die folgenden Beispiele dienen der Erläuterung des Verfahrens, ohne das Verfahren auf diese zu beschränken.

### Beispiel 1 (Vergleichsbeispiel):

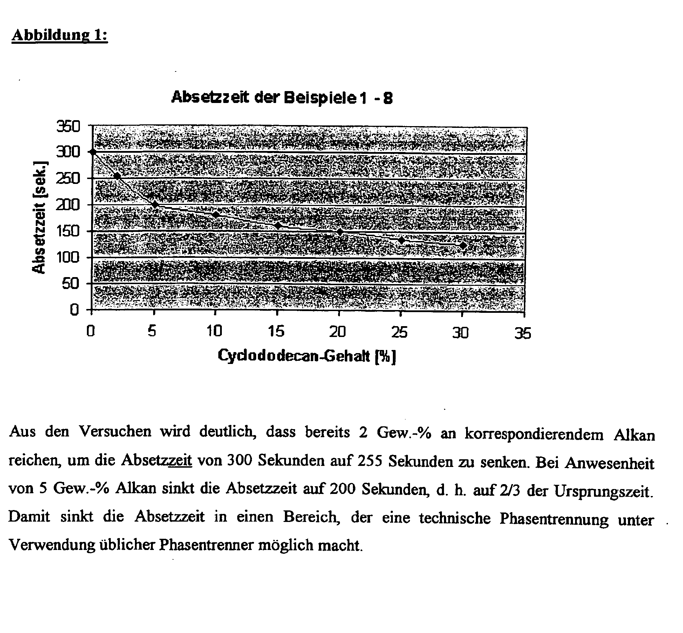

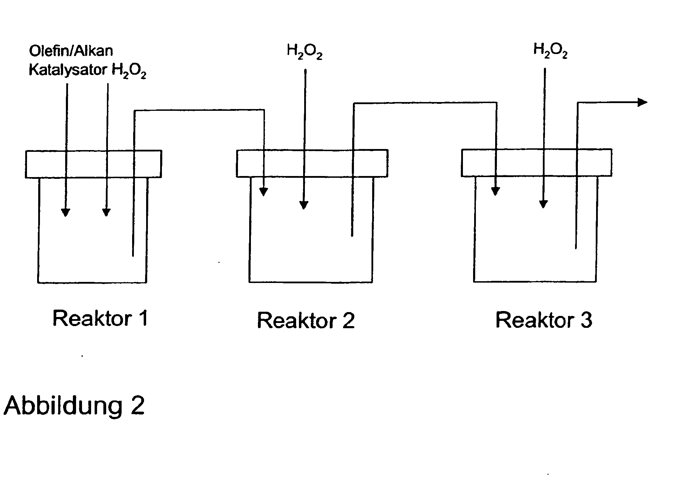

In einer Apparatur analog Abbildung 2 wurden in Reaktor 1 249 g Cyclododecen (1,5 Mol), 0,99 g Natriumwolframat, 0,59 g Phosphorsäure, 14 g Wasser, 1,2 g Aliquat 336 (Cognis) und 10,2 g Wasserstoffperoxid vorgelegt und mit Schwefelsäure auf pH 3 gebracht. Dann wurde auf 90 °C aufgeheizt und über 2 Stunden 102 g 50 %-ige Wasserstoffperoxidlösung zudosiert. In Reaktor 1 wurde ein Umsatz des Cyclododecens von etwa 90 % erreicht. Das Volumen betrug etwa 300 ml. Von nun an wurde kontinuierlich über 4 Stunden 2,7 ml/min einer Mischung aus Cyclododecen mit 0,2 Mol-% Aliquat 336 und mit 0,73 ml/min eine Lösung aus 0,94 % Natriumwolframat, 0,56 % Phosphorsäure und 98,5 % Wasserstoffperoxid (50 %-ig) zudosiert. Über einen Überlauf gelangte die Mischung kontinuierlich in Reaktor 2, der ebenfalls ein Volumen von 400 ml hatte und bei 90 °C betrieben wurde. Nach vier Stunden kontinuierlicher Fahrweise erreichte der Umsatz im Reaktor 3 99,6 %.

Nach der Reaktion wurde die Absetzzeit der Mischung in Reaktor 3 bestimmt. Die Absetzzeit ist die Zeit, nach der die wässrige Phase so abgesetzt war, das die zweiphasige Schicht am Rand des Reaktors kleiner als 1 mm betrug. Dabei wurde die Absetzzeit alle 15 Sekunden kontrolliert. Das Bestimmen der Absetzzeit wurde 2 Mal wiederholt und ein Mittelwert gebildet. Die Ergebnisse sind in Tabelle 1 dargestellt.

### Beispiel 2 (erfindungsgemäßes Beispiel):

Der Versuch wurde analog Beispiel 1 durchgeführt, nur wurde dem Edukt 2 % Cyclododecan bezogen auf Cyclododecen zugegeben. Die Ergebnisse sind in Tabelle 1 dargestellt.

### Beispiel 3 (erfindungsgemäßes Beispiel):

Der Versuch wurde analog Beispiel 1 durchgeführt, nur wurde dem Edukt 5 % Cyclododecan bezogen auf Cyclododecen zugegeben. Die Ergebnisse sind in Tabelle 1 dargestellt.

### Beispiel 4 (erfindungsgemäßes Beispiel):

Der Versuch wurde analog Beispiel 1 durchgeführt, nur wurde dem Edukt 10 % Cyclododecan bezogen af Cyclododecen zugegeben. Die Ergebnisse sind in Tabelle 1 dargestellt.

### Beispiel 5 (erfindungsgemäßes Beispiel):

Der Versuch wurde analog Beispiel 1 durchgeführt, nur wurde dem Edukt 15 % Cyclododecan bezogen auf Cyclododecen zugegeben. Die Ergebnisse sind in Tabelle 1 dargestellt.

### Beispiel 6 (erfindungsgemäßes Beispiel):

Der Versuch wurde analog Beispiel 1 durchgeführt, nur wurde dem Edukt 20 % Cyclododecan bezogen auf Cyclododecen zugegeben. Die Ergebnisse sind in Tabelle 1 dargestellt.

### Beispiel 7 (erfindungsgemäßes Beispiel):

Der Versuch wurde analog Beispiel 1 durchgeführt, nur wurde dem Edukt 25 % Cyclododecan bezogen auf Cyclododecen zugegeben.

### Beispiel 8 (erfindungsgemäßes Beispiel):

Der Versuch wurde analog Beispiel 1 durchgeführt, nur wurde dem Edukt 30 % Cyclododecan bezogen auf Cyclododecen zugegeben.

**Tabelle 1:**

| Beispiel Nr. | Cyclododecangehalt [in Gew.-%] | Absetzzeit [in Sekunden] |
|---|---|---|
| 1 (Vergleich) | 0 | 300 |
| 2 | 2 | 255 |
| 3 | 5 | 200 |
| 4 | 10 | 180 |
| 5 | 15 | 160 |
| 6 | 20 | 150 |
| 7 | 25 | 135 |
| 8 | 30 | 125 |

## Patentansprüche

1. Verfahren zur Epoxidierung von cyclischen, mindestens einfach ungesättigten Alkenen mit 8 bis 20 Kohlenstoffatomen im Cyclus in einem Reaktionsgemisch, das mindestens zwei flüssige Phasen enthält, in Gegenwart einer Peroxoverbindung als Oxidationsmittel und eines heterogenen und/oder homogenen Katalysators,
**dadurch gekennzeichnet,**
**dass** die Reaktionsmischung mindestens 1 Gew.-% des korrespondierenden gesättigten Alkans enthält.

2. Verfahren zur Epoxidierung von cyclischen, mindestens einfach ungesättigten Alkenen in Gegenwart eines Oxidationsmittels,
**dadurch gekennzeichnet,**
**dass** die Reaktionsmischung mindestens 2,5 Gew.-% des korrespondierenden gesättigten Alkans enthält.

3. Verfahren gemäß Anspruch 1 oder 2,
**dadurch gekennzeichnet,**
**dass** das heterogene Verfahren kontinuierlich ist.

4. Verfahren nach einem der Ansprüche 1 bis 3,
**dadurch gekennzeichnet,**
**dass** das Oxidationsmittel Wasserstoffperoxid ist.

5. Verfahren nach einem der Ansprüche 1 bis 4,
**dadurch gekennzeichnet,**
**dass** es in Gegenwart eines Katalysators, der mindestens ein Metall der IVb, Vb oder VIb des Periodensystems der Elemente darstellt oder enthält, oder in Gegenwart einer Mischung dieser Katalysatoren durchgeführt wird.

6. Verfahren nach einem der Ansprüche 1 bis 5,
**dadurch gekennzeichnet,**
**dass** die Reaktionsmischung mindestens einen Phasentransferkatalysator und Phosphorsäure enthält.

7. Verfahren gemäß Anspruch 6,
**dadurch gekennzeichnet,**
**dass** der Phasentransferkatalysator mindestens ein tertiäres Amin und/oder ein quartäres Ammoniumsalz enthält.

8. Verfahren nach einem der vorhergehenden Ansprüche,
**dadurch gekennzeichnet,**
**dass** der pH-Wert des Reaktionsgemisches zwischen 2 und 6, bevorzugt zwischen 2,5 und 4 gehalten wird.

9. Verfahren nach einem der vorhergehenden Ansprüche zur Herstellung von cyclischen Epoxyalkanen.

10. Verfahren nach Anspruch 9 zur Herstellung von 1,2-Epoxycyclododecan.

## Claims

1. Process for the epoxidation of cyclic, at least monounsaturated alkenes having from 8 to 20 carbon atoms in the ring in a reaction mixture which comprises at least two liquid phases in the presence of a peroxo compound as oxidant and a heterogeneous and/or homogenous catalyst, **characterized in that** the reaction mixture contains at least 1% by weight of the corresponding saturated alkane.

2. Process for the epoxidation of cyclic, at least monounsaturated alkenes in the presence of an oxidant, **characterized in that** the reaction mixture contains at least 2.5% by weight of the corresponding saturated alkane.

3. Process according to Claim 1 or 2, **characterized in that** the heterogeneous process is carried out continuously.

4. Process according to any of Claims 1 to 3, **characterized in that** the oxidant is hydrogen peroxide.

5. Process according to any of Claims 1 to 4, **characterized in that** it is carried out in the presence of a catalyst consisting of or comprising at least one metal of groups IVb, Vb and VIb of the Periodic Table of the Elements or in the presence of a mixture of such catalysts.

6. Process according to any of Claims 1 to 5, **characterized in that** the reaction mixture comprises at least one phase transfer catalyst and phosphoric acid.

7. Process according to Claim 6, **characterized in that** the phase transfer catalyst comprises at least one ternary amine and/or a quaternary ammonium salt.

8. Process according to any of the preceding claims, **characterized in that** the pH of the reaction mixture is kept in the range from 2 to 6, preferably from 2.5 to 4.

9. Process according to any of the preceding claims for preparing cyclic epoxyalkanes.

10. Process according to Claim 9 for preparing 1,2-epoxycyclododecane.

## Revendications

1. Procédé pour l'époxydation d'alcènes cycliques, au moins monoinsaturés, ayant 8 à 20 atomes de carbone dans le cycle, dans un mélange réactionnel qui contient au moins deux phases liquides, en présence d'un composé péroxo servant d'oxydant, et d'un catalyseur hétérogène et/ou homogène, **caractérisé en ce que** le mélange réactionnel contient au moins 1 % en poids de l'alcane saturé correspondant.

2. Procédé pour l'époxydation d'alcènes cycliques, au moins monoinsaturés, en présence d'un oxydant, **caractérisé en ce que** le mélange réactionnel contient au moins 2,5 % en poids de l'alcane saturé correspondant.

3. Procédé selon la revendication 1 ou 2, **caractérisé en ce que** le procédé hétérogène est continu.

4. Procédé selon l'une des revendications 1 à 3, **caractérisé en ce que** l'oxydant est le peroxyde d'hydrogène.

5. Procédé selon l'une des revendications 1 à 4, **caractérisé en ce qu'**il est mis en oeuvre en présence d'un catalyseur qui représente ou contient au moins un métal du groupe IVb, Vb ou VIb du Tableau Périodique des Eléments, ou en présence d'un mélange de ces catalyseurs.

6. Procédé selon l'une des revendications 1 à 5, **caractérisé en ce que** le mélange réactionnel contient au moins un catalyseur de transfert de phase et de l'acide phosphorique.

7. Procédé selon la revendication 6, **caractérisé en ce que** le catalyseur de transfert de phase contient au moins une amine tertiaire et/ou un sel de l'ammonium quaternaire.

8. Procédé selon l'une des revendications précédentes, **caractérisé en ce que** le pH du mélange réactionnel est maintenu entre 2 et 6 et de préférence entre 2,5 et 4.

9. Procédé selon l'une des revendications précédentes, pour préparer des époxyalcanes cycliques.

10. Procédé selon la revendication 9, pour préparer le 1,2-époxycyclododécane.
